Europäisches Patentamt

European Patent Office

Office européen des brevets

(11) Veröffentlichungsnummer: **0 461 568 A1**

# (12) EUROPÄISCHE PATENTANMELDUNG

(21) Anmeldenummer: **91109422.5**

(51) Int. Cl.⁵: **A61B 17/34**

(22) Anmeldetag: **08.06.91**

(30) Priorität: **11.06.90 DE 4018588**

(43) Veröffentlichungstag der Anmeldung:
**18.12.91 Patentblatt 91/51**

(84) Benannte Vertragsstaaten:
**AT BE DE DK ES FR GB IT NL**

(71) Anmelder: **Richard Wolf GmbH**
**Pforzheimer Strasse 32**
**W-7134 Knittlingen(DE)**

(72) Erfinder: **Hiltebrandt, Siegfried**
**August-Lämmle-Strasse 18**
**W-7134 Knittlingen(DE)**

(74) Vertreter: **Vollmann, Heiko, Dipl.-Ing. et al**
**Patentanwälte Wilcken & Wilcken,**
**Musterbahn 1**
**W-2400 Lübeck 1(DE)**

(54) **Trokarhülse.**

(57) Die Erfindung betrifft eine Trokarhülse zum Durchführen von gegen einen Trokar austauschbaren Endoskopen und Instrumenten, wobei der Schaft (1) der Trokarhülse proximal mit einem Handhabenteil (4) verbunden und verschließbar ist. Die proximale Stirnfläche des Handhabenteils (4) wird von einem Verschlußteil (6) abgedeckt, der von Hand quer zur Schaftachse verstellt werden kann und wenigstens einen Durchbruch (10) mit einem daran anschließenden Einführungsstutzen (11) aufweist. Der bzw. jeder Durchbruch (10) des Verschlußteils (6) kann durch dessen entsprechende Verdrehung mit dem Schaftkanal (2) in Überdeckung gebracht werden.

FIG. 1

EP 0 461 568 A1

Die Erfindung geht aus von einer Trokarhülse zum Durchführen von gegen einen Trokar austauschbaren Endoskopen und Operationsinstrumenten, wobei der Schaft der Trokarhülse proximal mit einem Handhabenteil verbindbar und gasdicht verschließbar ist.

Bekannte Trokarhülsen weisen üblicherweise Kugel-, Trompeten- oder Magnetventile auf, um den zentralen Schaftkanal der Trokarhülse vor dem Einführen oder nach dem Herausziehen eines durchgeführten Endoskopes, eines Hilfsinstrumentes oder dergleichen gasdicht zu verschließen. Solche Trokarhülsen sind in folgenden Literaturstellen offenbart:

Deutsche Gebrauchsmuster Nr. 70 04 051, 72 20 273, 74 30 345, 77 35 963 und 77 36 389.

Nachteilig bei den bekannten Trokarhülsen ist das durch eine Feder vorgespannte Verschlußelement, wodurch die Endoskope oder Instrumente an den Außenflächen angegriffen werden und wodurch die ungebremste Durchführung von Endoskopen oder Instrumenten verhindert wird. Ein Angriff der Instrumente kann deren Funktionsfähigkeit nachteilig beeinflussen oder die Instrumente sogar unbrauchbar machen. Da Trokarhülsen im allgemeinen aus Metall bestehen, kann sich dies bei Röntgenaufnahmen störend auswirken, da Organe des Körpers durch die Hülse abgedeckt werden können. Beim Instrumentenwechsel können während des Einführens oder der Entnahme aus Gasverluste auftreten.

Die Aufgabe der Erfindung besteht darin, die vorerwähnten Nachteile bekannter Trokarhülsen zu vermeiden, Endoskope und Instrumente ungehindert ohne Reibung durch die Trokarhülse in die Körperhöhle einführen zu können und Instrumente und Endoskope unterschiedlichen Durchmessers oder Querschnittprofils zur Anwendung bringen zu können.

Diese Aufgabe wird durch die Merkmale des Anspruches 1 gelöst. Weitere vorteilhafte Ausbildungen sind durch die Merkmale der Unteransprüche gekennzeichnet.

Die Erfindung wird nachstehend anhand der Zeichnung erläutert. Es zeigen:

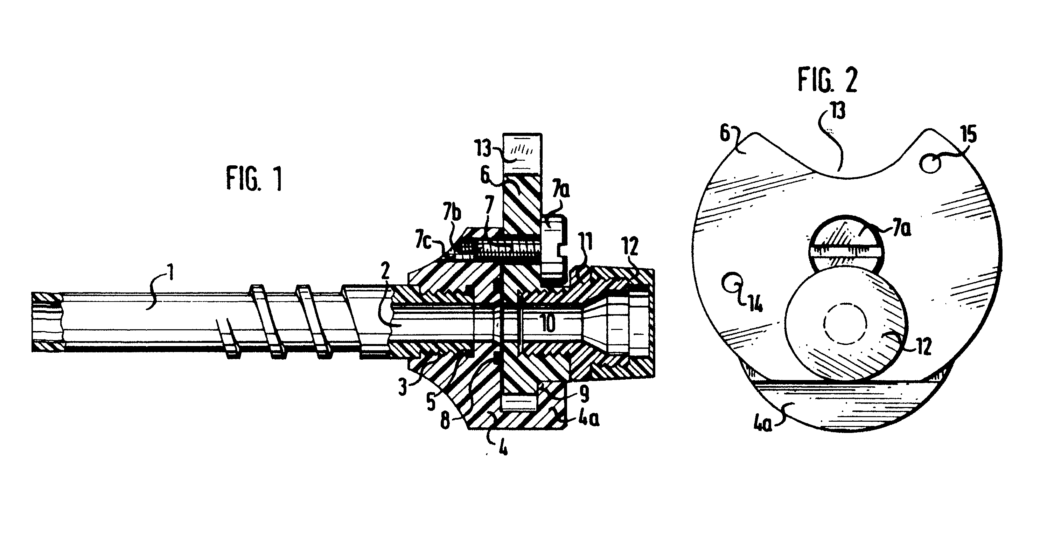

Figur 1     eine Seitenansicht einer Trokarhülse nach der Erfindung mit proximalem und distalem Axialschnitt,

Figur 2     eine Ansicht in Richtung gegen das proximale Ende der Trokarhülse nach Figur 1.

Die Trokarhülse nach der Erfindung besteht aus einem im Durchmesser sich proximalwärts etwas erweiternden Schaft 1 mit zentralem Kanal 2 und der mit dem Schaft 1 durch eine Verschraubung 3 verbindbaren Handhabe 4. Die Abdichtung zwischen dem proximalen Schaftende und dem Handhabenteil 4 wird durch einen eingelegten Dichtungsring 5 hergestellt.

Gegen die proximale Stirnfläche des Handhabenteils 4 liegt eine Verschlußscheibe 6. Diese Verschlußscheibe 6 wird mit geringem Spiel von einem zur Schaftachse parallelen Zapfen 7 durchgriffen, der durch einen Schraubkopf 7a mit einem Gewindeende 7b in eine Gewindebohrung 7c des Handhabenteiles 4 einschraubbar ist, wodurch die Verschlußscheibe 6 dichtend gegen die Stirnfläche des Handhabenteils gehalten wird. Die einwandfreie Abdichtung erfolgt durch einen in eine Ringnut des Handhabenteiles 4 eingelegten Dichtungsring 8. Um eine möglichst gleichmäßige Anlage der Verschlußscheibe 6 an der Stirnfläche des Handhabenteiles 4 zu erreichen, ist der Handhabenteil 4 auf der dem Befestigungszapfen 7 gegenüberliegenden Seite mit einem Ansatz 4a versehen, der gegen eine Schulter 9 der Verschlußscheibe 6 anliegt.

Die Verschlußscheibe 6 ist um den Zapfen 7 in zwei Richtungen verdrehbar, so daß ihr Durchbruch 10 durch Verdrehung der Verschlußscheibe 6 in eine Richtung mit dem Schaftkanal 2 in Überdeckung bringbar ist. An den Durchbruch 10 schließt sich ein Einführungsstutzen 11 für die durchzuführenden Instrumente an, auf den eine Dichtkappe 12 aufgesetzt ist.

Die mit einem Umfangsausschnitt 13 für den Eingriff eines Fingers versehene Verschlußscheibe 6 ist im und gegen den Uhrzeigersinn in zwei Stellungen verdrehbar. Im Uhrzeigersinn wird die Scheibe 6 verdreht, bis ein schaftseitiger Zapfen 15 der Scheibe 6 gegen den Umfang des Handhabenteils 4 anschlägt. Dann ist der Schaftkanal 2 durch die Verschlußscheibe 6 gasdicht verschlossen. Im umgekehrten Drehsinn kommt ein Zapfen 14 gegen den Umfang des Handhabenteils 4 zur Anlage. In dieser Anschlagstellung befindet sich der Durchbruch 10 der Verschlußscheibe mit dem Schaftkanal 2 in Überdeckung, so daß in dieser Stellung Instrumente durch die Trokarhülse in eine Körperhöhle eingeführt werden können.

Es ist auch möglich, die Verschlußscheibe 6 mit mehreren Durchbrüchen 10 unterschiedlichen Durchmessers und entsprechenden Einführungsstutzen zu versehen, um z.B. neben einem Durchbruch für Instrumente Adaptionselemente anzubringen, an die eine Endoskopkamera oder auch ein Druckmeßgerät oder dergleichen anschließbar ist.

Anstelle der verdrehbaren Verschlußscheibe kann auch ein Verschlußschieber zur Anwendung kommen, der von Hand quer zur Schaftachse an der proximalen Fläche des Handhabenteils geführt verschiebbar ist und in eine den Schaftkanal abschließenden Stellung sowie in eine oder mehrere Stellungen verschiebbar ist, in denen ein einzelner Durchbruch von mehreren Durchbrüchen mit dem Schaftkanal in Überdeckung bringbar ist. Diese Stellungen können durch Anschläge festgelegt sein.

**Patentansprüche**

1. Trokarhülse zum Durchführen von gegen einen Trokar austauschbaren Endoskopen und Instrumenten, deren Schaft proximal mit einem Handhabenteil verbunden und verschließbar ist, dadurch gekennzeichnet, daß die proximale Stirnfläche des Handhabenteiles (4) von einem Verschlußteil (6) abgedeckt ist, der von Hand quer zur Schaftachse verstellbar ist und mindestens einen Durchbruch (10) mit einem proximal anschließenden Einführungsstutzen (11) aufweist, und daß der Durchbruch mit dem Schaftkanal (2) in Überdeckung bringbar ist.

2. Trokarhülse nach Anspruch 1, dadurch gekennzeichnet, daß der Verschlußteil (6) als Scheibe ausgebildet und um einen zur Schaftachse parallelen Befestigungszapfen (7) des Handhabenteiles (4) so verdrehbar ist, daß die Verschlußscheibe (6) mit einem Durchbruch (10) mit dem Schaftkanal (2) fluchtet.

3. Trokarhülse nach Anspruch 1 und 2, dadurch gekennzeichnet, daß der Verschlußteil (6) auf der distalen Seite mit zwei Anschlagzapfen (14,15) versehen ist, die bei Verdrehung der Scheibe (6) gegen den Umfang des Handhabenteils (4) zur Anlage kommen und die Endstellungen des Verschlußteils bestimmen.

4. Trokarhülse nach einem der Ansprüche 1 bis 3, dadurch gekennzeichnet, daß der Verschlußteil (6) auf einer Seite des Schaftkanals (2) durch den Befestigungszapfen (7) gegen die proximale Stirnseite des Handhabenteils (4) gehalten ist und auf der dem Schaftkanal (2)-gegenüberliegenden Seite durch einen Ansatz (4a) des Handhabenteils (4) gegen die proximale Stirnseite des Handhabenteils (4) gehalten ist.

5. Trokarhülse nach einem der Ansprüche 1 bis 4, dadurch gekennzeichnet, daß der Handhabenteil (4) gegen den Verschlußteil (6) durch einen in eine Ringnut des Handhabenteiles eingelegten Dichtungsring (8) abgedichtet ist.

6. Trokarhülse nach einem der Ansprüche 1 bis 5, dadurch gekennzeichnet, daß der Verschlußteil (6) zwischen den beiden Anschlagzapfen (14,15) mit einer Umfangsausnehmung (13) für den Fingereingriff versehen ist.

7. Trokarhülse nach Anspruch 1, dadurch gekennzeichnet, daß der Verschlußteil als Schieber mit einem Durchbruch mit anschließendem Einführungsstutzen versehen ist und gegen die proximale Stirnfläche des Handhabenteiles quer geführt gehalten ist, wobei die Verschiebung in entgegengesetzte Richtungen durch Anschläge begrenzt ist, wobei bei Anschlaglage des Schiebers der Schaftkanal entweder verschlossen ist oder mit dem Durchbruch des Schiebers fluchtet.

FIG. 1

FIG. 2

EP 0 461 568 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁴) |
|---|---|---|---|
| X | <u>US - A - 4 112 932</u><br>(R.D. CHIULLI)<br>   * Gesamt; insbesondere Fig.<br>   1-3; Spalte 3, Zeile 22 -<br>   Spalte 4, Zeile 37 * | 1,2,5 | A 61 B 17/34 |
| A |  | 3,4,6 |  |
| A | <u>GB - A - 1 356 386</u><br>(NAT. RES. DEV. C.)<br>   * Fig. 1; Seite 2, Zeilen<br>   13-16 * | 1,7 |  |
| A | <u>AT - B - E 40 640</u><br>(ENDOTHERAPEUTICS C.)<br>   * Fig. 6,7,8,10; Seite 8,<br>   letzter Absatz - Seite 11,<br>   3. Absatz * | 1,7 |  |

RECHERCHIERTE SACHGEBIETE (Int. Cl⁴)

A 61 B

Der vorliegende Recherchenbericht wurde für alle Patentansprüche erstellt.

| Recherchenort<br>WIEN | Abschlußdatum der Recherche<br>24-09-1991 | Prüfer<br>LUDWIG |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503 03 82